# EUROPEAN PATENT APPLICATION

(11) **EP 1 524 320 A1**
(43) Date of publication of application: **20.04.2005**
(21) Application number: 03023512.1
(22) Date of filing: 15.10.2003
(51) Int. Cl.: C12Q 1/68, C12N 5/06

(54) **Rapid determination and quantification of mycoplasma contamination using DNA chip technology**

(71) Applicant: Deutsches Krebsforschungszentrum Stiftung des öffentlichen Rechts, 69120 Heidelberg (DE)
(72) Inventor: Werner, Ekkehard, 69117 Heidelberg (DE)
(74) Representative: Schuck, Alexander

(57) **Abstract**

Disclosed is a method for detecting a microorganism contamination in a culture of eukaryotic cells to be used for gene expression profiling, by using a microarray which has attached on its surface at least one nucleic acid probe representing a gene of a eukaryotic cell and at least one nucleic acid probe representing a gene of a microorganism, e.g. mycoplasma.

SEQUENCE LISTING

<110>  Deutsches Krebsforschungszentrum (DKFZ)

<120>  Rapid determination and quantification of mycoplasma contamination using
DNA chip technology

<130>  DK62217EP

<160>  5

<170>  PatentIn version 3.1

<210>  1
<211>  20
<212>  DNA
<213>  Mycoplasma

<400>  1
acaccatggg agctggtaat                                                    20

<210>  2
<211>  20
<212>  DNA
<213>  T7 Phage

<400>  2
taatacgact cactataggg                                                    20

<210>  3
<211>  27
<212>  DNA
<213>  Mycoplasma

<220>
<221>  misc_feature
<222>  (5)..(5)

<223>  "a" or "t"

<220>

<221>  misc_feature

<222>  (13)..(13)

<223>  "t" or "c"

<400>  3
cttcntcgac ttncagaccc aaggcat                                          27

<210>  4

<211>  18

<212>  DNA

<213>  Mycoplasma

<400>  4
gttctttgaa aactgaat                                                    18

<210>  5

<211>  18

<212>  DNA

<213>  Mycoplasma

<220>

<221>  misc_feature

<222>  (11)..(11)

<223>  "a" or "t"

<220>

<221>  misc_feature

<222>  (13)..(13)

<223>  "a" or "t"

<400>  5
gcatccacca nanactct                                                    18

## Description

The present invention relates to a method for mycoplasma detection using mycoplasma-specific nucleic acid primers for DNA chip hybridization.

The wide distribution of mycoplasma contaminations represents a commonly well known problem for maintaining cells in culture. The effects on the reproducibility of research results as well as the threatening potential of mycoplasma contaminations at the production of pharmaceuticals or the therapeutic cell replacement are largely underestimated. An efficient quality control with a rapid and reliable detection method becomes increasingly relevant for research and industrial applications.

The prokaryotic microorganisms with the trivial name "mycoplasma" belonging to the class of mollicutes comprise more than 150 different species. Mycoplasma possess a relatively small genome and are not able to synthesize all nutritions required for their growth. Thus, they commonly live as pathogenic parasites in close contact with eukaryotic host cells. Cell cultures offer mycoplasma necessarily ideal living conditions: sufficient amounts of nutritions and optimal temperatures at routineous care. Depending on various parameters, statistically 4 to 92 % of cultures of a cell culture laboratory are contaminated with mycoplasma. Even though mycoplasma in cell culture supernatants can increase to a density of 10⁷ to 10⁸/ml, a contamination by mycoplasma cannot be detected by viewing cultures. In addition, mycoplasma are not always detectable with macroscopic alteration of the cells or media. The effects on cell cultures, though, can be enormous: a remarkable inhibition of cell proliferation, influence on virus proliferation, induction or suppression of different functions of cell metabolism, etc. Furthermore, it has to be noticed that approximately 30 to 50 % of the protein and DNA material obtained from a cell culture can be of mycoplasm origin and, thus, affects as contaminations the further investigations.

Of particular relevance is the observation that mycoplasm contaminations can possibly alter the gene expression pattern of the infected host cell. On the other hand, is a stable gene expression in a cell culture a prerequisite to create a meaningful gene expression profile, for example, by employing microarray technologies.

Only a routineous testing of cell cultures protects from these deleterious effects. Mycoplasma can cause serious diseases for humans and animals, particularly of breathing organs and the urogenital tract. For the pharmaceutical production the mycoplasma detection is regulated by law. According to the European Pharmacopoeia (EUR. Ph. 2.6.7), production and control cells, virus stocks and the final product must be tested to be free from mycoplasma after a fixed scheme. These include culture procedures on agar, broth, or semi-solid agar-broth medium; propagation on susceptible indicator cell lines for detection of mycoplasma species that are not cultivable in cell-free medium; DNA-staining procedures with dyes like DAPI (4',6-diamino-2'-phenylindoldihydrochloride); biochemical identification methods that detect enzyme activities present in mycoplasma but absent or minimal in non-infected cell cultures; detection by specific fluorescein or peroxidase conjugated polyclonal antisera or monoclonal antibodies with fluorescence microscopy, enzyme-linked immunosorbent assays (ELISA, as in the Roche mycloplasma detection kit); and the use of the polymerase chain reaction (PCR) using mycoplasma-specific amplifying primers.

For example, US 5,693,467 discloses a method for detecting and identifying mycoplasma contaminations by performing nested PCR, a two-stage amplification method, with DNA derived from test samples by using Mycoplasma specific primers detecting Mycoplasma 16S rRNA regions. Furthermore, US 5,595,871 discloses methods for detecting Mycoplasma hominis in a sample by hybridization techniques which employs oligonucleotide primers specific for M. hominis.

Even though the methods of the prior art make it possible to detect mycoplasma contaminations in a higher number of samples and also to detect a variety of Mycoplasma species possibly present in a sample, these methods are not suitable to be directly applied together with microarray formats in order to analyze the gene expression of the cell culture of interest.

Thus, the problem underlying the present invention is the provision of a method to detect contaminations of microorganisms, in particular mycoplasma, in cell cultures the cellular content of which should be subjected to gene expression profiling.

The problem is solved by a method for detecting a microorganism contamination in a culture of eukaryotic cells to be used for gene expression profiling, the method comprising:
(a) providing a microarray which has attached on its surface
   (a.1) at least one nucleic acid probe representing a gene of a eukaryotic cell and
   (a.2) at least one nucleic acid probe representing a gene of a microorganism,
(b) preparing a nucleic acid target sample from the cell culture by means of a primer mixture suitable for amplifying said at least one gene of a eukaryotic cell and said at least one gene of a microorganism,
(c) contacting the microarray from (a) with the nucleic acid target sample from (b) to permit selective hybridization between the nucleic acid targets and their nucleic acid probes on the microarray and
(d) detecting said hybridization thereby detecting a microoorganism contamination and/or detecting the expression of genes specific for the eukaryotic cell.

Additionally, the method can comprise a further step
(e) comparing the gene expression pattern of contaminated eukaryotic cells with the gene expression pattern non-contaminated eukaryotic cells.

With the method subject to the present invention, it is possible to detect the presence of a microorganism in a cell culture by parallel hybridizing nucleic acid probes specific for the genome of said eukaryotic cells and nucleic acid probes specific for the genome of the contaminating microorganism by employing microarray technology as described hereinafter.

The term "microorganism" refers to a small unicellular organism with dimension beneath the limits of vision which can be propagated and manipulated in a laboratory. In the context of the present invention, "microorganism" means prokaryotes like viruses, bacteria, cyanobacteria, Mollicutes and unicellular eukaryotes (protozoa) like certain genera of fungi, algae and parasites (e.g. sporozoa), and does not mean cultured cells of multicellular eukaryotes (metazoa).

The "culture of eukaryotic cells" are ideally singularized, cultured metazoan cells of plants, insects, birds (e.g. chicken, turkey, goose, dove), mammals (e.g. human, cattle, horse, sheep, goat, pig, dog, cat, rat, mouse, guinea pig). In a preferred embodiment of the present invention the eukaryotic cells are mammalian cells, more preferably human cells.

The term "gene expression profiling" refers to the simultaneous measurement of at least two, preferably at least twenty, more preferably at least hundred, and most preferred thousands of genes at a specific moment in time. This hybridization-based analysis of expression of genes in given cells/tissues tells where, when, and to what extent a particular gene is expressed and which physiological pathways are active in the cell. By comparing profiles, the pattern by which a gene is expressed can provide clues as to a gene's function in a particular biological process or pathway.

Essential for the method of the present invention is the provision of a "microarray", which has attached on its surface at least one nucleic acid probe representing a gene of a microorganism and at least one nucleic acid probe representing a gene of a eukaryotic cell. In general, a "microarray" refers to a linear or two-dimensional arrangement of preferably discrete nucleic acid probes / nucleic acid library which comprises an intentionally created collection of nucleic acid probes of any length spotted onto a substrate/solid support. The person skilled in the art knows a collection of nucleic acids spotted onto a substrate/solid support also under the term "array". As known to the person skilled in the art, a microarray usually refers to a miniaturised array arrangement, with the probes being attached to a density of at least about 100 nucleic acid molecules referring to different or the same genes per cm². Furthermore, where appropriate an array can be referred to as "gene chip". The array itself can have different formats, e.g. libraries of soluble nucleic acid probes or libraries of probes tethered to resin beads, silica chips, or other solid supports.

The molecules, in particular the "nucleic acid probes", on an array can be obtained synthetically or biosynthetically. The sequence and/or the concentration of the nucleic acid probe on the array can be identical or different from each other. The "nucleic acid probe" is generally a nucleic acid sequence representing a gene or at least a part of a gene either of the eukaryotic cell culture or of the microorganism which is attached onto a solid support. This can occur either by spotting PCR products on solid supports, or by direct chemical synthesis on the solid support using photolithography. The nucleic acid probe can be a polymeric form of nucleic acids, either ribonucleotides, deoxyribonucleotides or peptide nucleic acids (PNAs).

In general, the solid support onto which the nucleic acid probes are spotted and attached refers to a material with a rigid or semi-rigid surface which can assume a variety of forms and configurations (e.g. flat form, wells, beads, resins, gels, and the like). The solid support or substrate onto which the nucleic acid probes are spotted and attached can be, for example, a filter or membrane array consisting preferably of Nylon. Such arrays usually expose on their surface several hundreds of nucleic acid probes usually having a length of 500 to 2000 bases. Furthermore, it is also possible that the solid support is a so-called high-density microarray whereby the substrate is typically a non-porous material such as a plastic material, glass or silicon. Preferably, the solid support consists of silicon. High-density arrays usually expose on their surface several thousands of nucleic acid probes usually having a length of up to 100 bases.

The process of array fabrication is well-known to the person skilled in the art. Commonly, the process comprises preparing a glass (or other) slide (e.g. chemical treatment of the glass to enhance binding of the nucleic acid probes to the glass surface), obtaining DNA sequences representing genes of a genome of interest, and spotting sequences these sequences of interest onto glass slide. Sequences of interest can be obtained via creating a cDNA library from an mRNA source or by using publicly available databases, such as GeneBank, to annotate the sequence information of custom cDNA libraries or to identify cDNA clones from previously prepared libraries. Generally, it is recommendable to amplify obtained sequences by PCR in order to have sufficient amounts of DNA to print on the array. The liquid containing the amplified probes can be deposited on the array by using a set of microspotting pins. Ideally, the amount deposited should be uniform. The process can further include UV-crosslinking in order to enhance immobilization of the probes on the array.

The terms "target" or "nucleic acid target" refer to nucleic acid sequences, which are, on one hand, specific for the genome of the microorganism to be detected, and, on the other hand specific for the culture of eukaryotic cells to be subjected to gene expression profiling. These nucleic acid sequences include the original nucleic acid sequence to be amplified, the complementary second strand of the original nucleic acid sequence to be amplified and either strand of a copy of the original sequence which is produced by the amplification reaction. These copies serve as amplifiable targets by virtue of the fact that they contain copies of the sequence to which the primer as described below hybridizes.

The nucleic acid targets are prepared after methods known to the person skilled in the art. The sample preparation, in general, consists of processing and preparing the biological sample of interest, which includes isolating total or mRNA from a cell culture, reverse transcription of the RNA into cDNA, amplification of said cDNA by polymerase chain reaction (PCR) or by in vitro transcription by means of suitable primers hybridizing to specific target regions within the RNA and labelling of nucleic acid target samples. If the cDNA is amplified by in vitro transcription the obtained cRNA has to be fragmented afterwards according to, e.g. the Affymetrix expression analysis manual, which is incorporated herein by reference.

The labelling of the nucleic acid sample can occur during or after reverse transcription or PCR. The label can be luminescent or radioactive, with luminescent labels being preferred. Suitable compounds for labelling nucleic acids include digoxygenin, radioactively labelled nucleotides, biotin or biotinylated nucleotides, fluorescent dyes such as Cyanine-3, Cyanine-5, fluoresceine, tetramethylrhodamine, and BODIPY, or variants thereof. The person skilled in the art knows a variety of methods employed in labelling of nucleic acids, all of which are included in the present invention. Suitable methods include the direct labelling (incorporation) method, the amino-modified (amino-allyl) nucleotide method, and the primer tagging method (DNA dendrimer labelling).

As used herein, the "primer mixture" used to prepare the nucleic acids targets represents short pre-existing DNA or RNA fragments (primers) which can be annealed to single-stranded DNA, from which DNA polymerase extends a new DNA strand to produce a duplex molecule by adding new deoxyribonucleotides. A primer of the primer mixture acts as the initiation point for template-directed nucleic acid synthesis in the presence of four nucleoside triphopsphates and a polymerization agent (e.g. DNA, RNA polymerase, reverse transcriptase). The length of the primer usually determines the hybridization temperature, with shorter primers requiring cooler temperatures for the formation of a stable hybrid complex with the template. Generally, the preferred length of a primer comprises 15-20, 25, 30 nucleotides.

The length of the primers of the primer mixture for use in detecting a cell culture contaminating microoorganism, and, optionally, for use in gene expression profiling, depends on several factors including the nucleotide sequence and the temperature at which these nucleic acids are hybridized. The considerations necessary to determine a preferred length for a primer are well known to the skilled artisan. The length of a short nucleic acid or oligonucleotide can relate to its hybridization specificity or selectivity. When a test sample contains complex mixtures of nucleic acids, e.g. genomic DNA of the eukaryotic cells together with the DNA of the microorganism, oligonucleotides which are shorter than about 14 nucleotides may hybridize to more than one site in the eukaryotic genome, and accordingly would not have sufficient hybridization selectivity for detecting a single target nucleic acid. However the sequence of a nucleic acid which is about 14-15 nucleotides is generally represented only once in a eukaryotic genome. Accordingly, to eliminate cross hybridization with eukaryotic genomic DNA of the cell culture, the primers of the present invention are generally at least about 14 nucleotides long. However, as is known to the skilled artisan nucleic acids or oligonucleotides which are shorter than 14 nucleotides, e.g. oligonucleotides of about 10 to about 12 or more nucleotides, can be specific for a given target. Therefore the term at least "about" is used to include any such nucleic acids and oligonucleotides which are less than 14 nucleotides long but which selectively hybridize to a microorganism target nucleic acid. Preferably, the present primers are at least 16 nucleotides in length. More preferred primers are at least 17 nucleotides in length.

For the present invention it is most suitable when the primer is synthesized chemically by means known to the person skilled in the art. Primers, e.g. of up to about 50 nucleotides, can be chemically synthesized by available synthetic procedures for nucleic acids. Chemical synthesis of nucleic acids is well known in the art and can be achieved by solution or solid phase techniques. Moreover, primers of defined sequence can be purchased commercially or can be made by any of several different synthetic procedures including the phosphoramidite, phosphite triester, H-phosphonate and phosphotriester methods, typically by automated synthesis methods. Modified bases can also be incorporated into the nucleic acid. If modified phosphodiester linkages are used the synthetic procedures are altered as needed according to known procedures (Uhlmann et al., 1990 Chemical Reviews 90:543-584).

Enzymatic methods are also available for DNA, RNA or oligonucleotide synthesis. For DNA and oligodeoxyribonucleotide synthesis, these methods frequently employ Klenow, T7, T4, Taq or E. coli DNA polymerases, e.g. as described in Sambrook et al. Enzymatic methods of RNA or oligoribonucleotide synthesis frequently employ SP6, T3 or T7 RNA polymerase as described, for example, in Sambrook et al. Reverse transcriptase can also be used to synthesize DNA from RNA. Preparing a primer enzymatically requires a template nucleic acid which can either be synthesized chemically, or be obtained as mRNA, genomic DNA, cloned genomic DNA, cloned cDNA or recombinant DNA. Some enzymatic methods of DNA or oligodeoxyribonucleotide synthesis can require a short primer oligonucleotide; this primer can be obtained or synthesized by any available procedure.

After enzymatic or chemical synthesis, primers can be purified by polyacrylamide gel electrophoresis, or by any of a number of chromatographic methods, including gel, ionexchange and high pressure liquid chromatography. To confirm a nucleotide sequence, primers can be subjected to DNA sequencing by available procedures, including Maxam and Gilbert sequencing, Sanger sequencing, capillary electrophoreses sequencing the wandering spot sequencing procedure or by using selective chemical degradation of oligonucleotides bound to Hybond paper.

For the method of the present invention it is preferred that the nucleic acid targets obtained by the procedures described above are contacted with a microarray having attached on its surface at least one nucleic acid probe representing a gene of a eukaryotic cell and at least one nucleic acid probe representing a gene of a microorganism, to permit a selective hybridization between the nucleic acid targets and their complementary nucleic acid probes on the microarray.

"Complementary" and "complementarity", respectively, can be described by the percentage, i.e. proportion, of nucleotides which can form base pairs between two nucleic acid strands or within a specific region or domain of the two strands. Generally, complementary nucleotides are, according to the base pairing rules, A and T (or A and U), and C and G. Complementarity may be partial, in which only some of the nucleic acids' bases are matched according to the base pairing rules. Or, there may be a complete or total complementarity between the nucleic acids. The degree of complementarity between nucleic acid strands has significant effects on the efficiency and strength of hybridization between nucleic acid strands. This is of particular importance in amplification and hybridization reactions, as well as detection methods that depend upon binding between nucleic acids.

Two nucleic acid strands are considered to be 100% complementary to each other over a defined length if in a defined region all As of a first strand can pair with a T (or an U) of a second strand, all Gs of a first strand can pair with a C of a second strand, all T (or Us) of a first strand can pair with an A of a second strand, and all Cs of a first strand can pair with a G of a second strand, and vice versa. According to the present invention, the degree of complementarity is determined over a stretch of 20 nucleotides, i.e. a 60% complementarity means that within a region of 20 nucleotides of two nucleic acid strands 12 nucleotides of the first strand can base pair with 12 nucleotides of the second strand according to the above ruling, either as a stretch of 12 contiguous nucleotides or interspersed by non-pairing nucleotides, when the two strands are attached to each other over said region of 20 nucleotides.

The degree of complementarity can range from at least about 50% to full, i.e. 100% complementarity. Two single nucleic acid strands are said to be "substantially complementary" when they are at least about 80% complementary, preferably about 90% or higher. Therefore, according to the present invention the degree of complementarity that the nucleic acid probes have with the nucleic acid targets need not be 100%, as long as selective hybridization to the microorganism can be achieved and detected. Selective hybridization can occur when there is at least about 65% complementarity over a stretch of 20 nucleotides, preferably at least about 75%, more preferably at least about 90%.

The term "selective hybridization" means that two nucleic acid strands which are substantially complementary, hybridize with each other, but not two strands with only a partial degree of complementarity, that is complementarity of less than 30% according to the definition above. In the absence of non-selective hybridization the probe will not bind to the non-complementary target, which can be used as a measure or reference for determining the degree of selective hybridization ("negative control").

Conditions suitable for permitting selective hybridization can be determined by the skilled person in the following way. Stringency hybridization conditions can be altered by varying the reaction parameters either individually or in concert. With "high stringency" conditions (providing in general high temperature, high formamide, low salt), nucleic acid base pairing will occur only between nucleic acid fragments that have a high frequency of complementary base sequences (e.g., with about 70-100% complementarity). With "medium or low stringency" conditions (providing in general low temperature, low formamide, high salt), nucleic acid base pairing will occur between nucleic acids with an intermediate or low frequency of complementary base sequences (e.g., hybridization under "medium stringency" conditions may occur between nucleic acid targets and nucleic acid probes with about 50-70% complementarity).

For the purpose of achieving selective hybridization, the skilled artisan is aware of a variety of hybridization conditions and stringencies (e.g. described in Sambrook et al., A laboratory Manual). The incubation conditions, e.g. time, salt concentrations, temperature, can vary and usually depend on the sequence and length of the prepared nucleic acid targets and the nucleic acid probes on the array and may therefore be adjusted each time. The conditions should be chosen at stringency to permit a selective hybridization between the target and the complementary probe, i.e. to allow selective hybridization occur while limiting non-selective hybridization.

Hybridizations can be performed at salt concentrations of no more than about 1 Molar (1M), preferably no more than 500mM, more preferably no more than 200mM, and the hybridization temperature is greater than 22 degree Celsius (22°C), preferably at least 25 degree Celsius (25°C), more preferably at least 30°C. However, longer nucleic acid targets/probes may require higher temperatures for selective hybridization. Hybridization reactions are preferably performed under stringency conditions which select for hybridizations between nucleic acids with a higher degree of complementary. Additional guidance regarding such conditions is readily available in the art, for example, by Sambrook et al., A Laboratory Manual, Cold Spring Harbor Press, N. Y.; and Ausubel et al., Current Protocols in Molecular Biology, (John Wiley & Sons, N. Y. ) Such conditions are, for example, hybridization in 6x SSC, pH 7.0 / 0.1% SDS at about 45°C for 18-23 hours, followed by a washing step with 2x SSC/0.1% SDS at 50°C. In order to select the stringency, the salt concentration in the washing step can for example be chosen between 2x SSC/0.1% SDS at room temperature for low stringency and 0.2x SSC/0.1% SDS at 50°C for high stringency. In addition, the temperature of the washing step can be varied between room temperature, ca. 22°C, for low stringency, and 65°C to 70° C for high stringency.

It will be recognized by those of skill in the art that the choice of stringency conditions (high, medium or low stringency) required for selective hybridization resulting in a stable hybrid will depend on several factors, for example: length and G/C content (melting temperature, as described below) of the nucleic acid strands to be hybridized, positioning of mismatched bases (if any), degree of uniqueness of the sequence as compared to the population of nucleic acid targets, presence or absence of a probe mixture or unique probes, and chemical nature of the nucleic acids to be hybridized (e.g., methylphosphonate backbone or phosphorothiolate), among others. In the following, an example as to how a skilled artisan can control hybridization stringency through hybridization and washing conditions is provided. Nucleic acid hybrid stability is expressed as the melting temperature or Tm, which is the temperature at which a probe dissociates from a target DNA, i.e. the temperature at which a population of double-stranded nucleic acid molecules becomes half dissociated into single strands. The equation for calculating the Tm of nucleic acids is well known in the art. As indicated by standard references, a simple estimate of the Tm value may be calculated by the equation: Tm=81.5+0.41(% G+C), when a nucleic acid is in aqueous solution at 1 M NaCl. This melting temperature is used to define the required stringency conditions. If sequences are to be identified that are related and substantially complementary to the probe, then it is useful to first establish the lowest temperature at which only homologous hybridization occurs with a particular concentration of salt (e.g., SSC or SSPE). Assuming that 1% mismatching results in a 1 degree Celsius decrease in the Tm, the temperature of the final wash in the hybridization reaction is reduced accordingly. In practice, the change in Tm can be between 0.5 degree C and 1.5 degree C per 1% mismatch.

In typical experiment, selective hybridization is achieved when the cDNA obtained by reverse transcribing the target mRNA of the sample is used in an in vitro transcription for 6hrs at 37 degree Celsius, the obtained cRNA is fragmented by incubation in fragmentation buffer, 20 microgram of the fragmented cRNA is hybridized to a GeneChip in 200 microliter hybridization buffer (100mM MES buffer, pH6.6, 1M NaCl, 20mM EDTA, 0.01 microgram hering sperm DNA (100 microgram/ml), acetyl-BSA (500 microgram/ml) and 0.01% Tween 20, for 16 hrs at 45 degree Celsius in a hybridization oven, followed by washes in low stringency buffer (6xSSPE, 0.01% Tween) at 25 degree Celsius and washes in high stringency buffer (100mM MES, 0.1M NaCl, 0.01 % Tween) at degree Celsius.

In view of the foregoing, the person of skill in the art is aware that the conditions and parameters required to achieve selective hybridization between a nucleic acid target and a nucleic acid probe will have to be adjusted according to the methods known in the art or presented herein for each particular case and microarray preparation. Appropriate positive and negative controls can be included in the experiments, if desired.

In order to detect a possible microorganism contamination and, optionally, the expression level of genes specific for the eukaryotic cell, the hybridization between the nucleic acid targets and their complementary nucleic acid probes on the microarray is detected by methods known to the person skilled in the art. Detection is enabled by the labelling of the nucleic acid targets prior to hybridization with the nucleic acid probes on the microarray by employing labelling methods as described supra. Accordingly, locations at which the nucleic acid targets hybridize with complementary probes on the microarray can be identified by locating the label. In principle, the qualitative and quantitative expression of a gene specific for the microorganism or for the eukaryotic cell is measured by the spot intensity of the corresponding label attached to the nucleic acid target which is hybridized to the nucleic acid probe on the microarray. Dye or fluorescent intensities are obtained by scanning the array using e.g. a confocal laser microscope. If the label is a radioactive label, for example ³²P, the hybridization can be detected by a phosphoimager.

If a microorganism contamination is detected, the method of the present invention can comprise an additional step, i.e. comparing the gene expression of contaminated eukaryotic cells with the gene expression of non-eukaryotic cells.

In a preferred embodiment of the present invention the microorganism to be detected by the method of the present invention belongs to the class of Mollicutes. The Mollicutes comprise the genera Mycoplasma, Ureaplasma, Entomoplasma, Acholeplasma (e.g. A. axanthum, A. laidlawii), Phytoplasma (mycoplasma-like organisms, MLOs) and Spiroplasma, with Mycoplasma being the most preferred genus. A variety of Mycoplasma species are known to the person skilled in the art and comprise species such as M. pneumoniae, M. pulmonis, M. genitalium, M. penetrans, M. gallisepticum, M. gallinarium, M. synoviae, M. fermentans, M. hyorhinis, M. arginini, M. orale, M. salivarium, M. hominis, M. arthritidis, M. bovis, M. pirum, M. capricolum, M. meleagridis, M. iowae, M. mycoides, M. anseris, M. columbinum, M. anatis, M. dispar, M. bovigenitalium, M. alkalescens, M. californicum, M. wenyonii, M. bovoculi, M. verecundum, M. canadense, M. alvi, M. agalactiae, M. putrefaciens, M. ovipneumoniae, M. conjunctivae, M. oculi, M. hypopneumoniae, M. flocculare, M. hyosynoviae, M. granularum, M. sualvi, M. haemosuis, M. felis, M. equirhinis, M. equipharyngis, M. equigenitalium, M. subdolum, M. equifetale, M. hippikon, M. canis, M. spumans, M. maculosum, M. opalescens, M. gatae, M. edwardii, M. molare, M. cynos, M. feliminutum, M. haemocanis, M. haemofelis, M. neurolyticum, M. caviae and M. haemomuris.

As a consequence, the primer mixture used for preparing nucleic acid samples contains at least one primer which comprises a sequence hybridizing to the 16S or 23S rRNA gene region of Mycoplasma, Ureaplasma, Entomoplasma, Acholeplasma, Phytoplasma and Spiroplasma, preferably a sequence as defined by SEQ ID NOs: 1, 3, 4, and 5 (see EXAMPLES). Most preferably, the primer hybridizing to the 16S rRNA comprises the sequence as defined by SEQ ID NO:1.

The primers of the primer mixture comprise a 3' target binding sequence which hybridizes to a complementary sequence in the target, e.g. the 16S or 23S rRNA of Mycoplasma, and can, optionally, further comprise a 5' tail sequence which is not complementary to the target, e.g. a promoter region. Generally, the promoter is a region of DNA usually (i.e. in vivo) extending 150-300 base pairs upstream from the transcription start site that contains binding sites for RNA polymerase and a number of proteins that regulate the rate of transcription of the adjacent gene. It represents a DNA site to which RNA polymerase will bind and initiate transcription. In the case of the present invention the promoter is ideally a short stretch of about 20 nucleotides representing the binding site for a RNA polymerase which is used for in vitro transcription, in particular the binding site for a phage RNA polymerase.

By using preferably a primer comprising a sequence which hybridizes to a complementary sequence in the target and a sequence of a phage promoter, the preparing of a nucleic acid target sample in step b) of the method of the present invention includes an in vitro transcription reaction for the amplification of the cDNA.

Transcription is the process by which RNA polymerase directs the synthesis of RNA from a DNA template under the control of a suitable promoter. In vitro transcription reactions use purified components to essentially mimic processes that occur in vivo. The principal components of a typical in vitro transcription reaction are the DNA template, RNA polymerase and ribonucleotide triphosphates (rNTPs). Depending on the type of the promoter, the RNA polymerase used for in vitro transcription can either be T7, T3 or SP6 polymerase.

The primer is, first, extended by a RNA dependent DNA polymerase, in particular reverse transcriptase. The promoter region within the primer, then, allows for in vitro transcribing the obtained cDNA into cRNA, followed by the synthesis of the second strand. Preferably, the promoter is a phage promoter allowing in vitro transcription. More preferably, the promoter is T3, T7, or SP6 promoter. Most preferred, the promoter comprises a sequence of the T7 promoter namely the sequence of TAATACGACTCACTATAGGG (SEQ ID NO:2).

To produce labelled high specific activity RNA targets the standard transcription reaction should include a (radio) labelled rNTP. Suitable isotopes are either 35S-labelled or 32P-labelled GTP, UTP or CTP. ATP is not recommended due to low incorporation kinetics. Preferably, the standard transcription reaction includes non-radioactively labelled rNTPs, more preferably, it includes biotinylated rNTPs.

In a further embodiment, the present invention refers to a microarray which has attached on its surface
(a.1) at least one nucleic acid probe representing a gene of a eukaryotic cell and
(a.2) at least one nucleic acid probe representing a gene of a microorganism

In a preferred embodiment, the array of the present invention which is used for detecting the presence of a microorganism in a cell culture is a high density oligonucleotide (oligo) array using a light-directed chemical synthesis process, employing the so-called photolithography technology. Unlike common cDNA arrays, oligo arrays (according to the Affymetrix technology) use a single-dye technology. Given the sequence information of the genes of the cell culture to be tested together with the sequence information of the target sequence of the potentially contaminating microorganism, e.g. 16S rRNA, the sequence can be synthesized directly onto the array, thus, bypassing the need for physical intermediates, such as PCR products, required for making cDNA arrays. For this purpose, the gene, e.g. the 16S rRNA gene of Mycoplasma, or partial sequences thereof, can be represented by 14 to 20 features, preferably by less than 14 features, more preferably less than 10 features, even more preferably by 6 features or less, with each feature being a short sequence of nucleotides (oligonucleotide), which is a perfect match (PM) to a segment of the respective gene. The PM oligonucleotide are paired with mismatch (MM) oligonucleotides which have a single mismatch at the central base of the nucleotide and are used as "controls". The chip exposure sites are defined by masks and are deprotected by the use of light, followed by a chemical coupling step resulting in the synthesis of one nucleotide. The masking, light deprotection, and coupling process can then be repeated to synthesize the next nucleotide, until the nucleotide chain is of the specified length.

In a more preferred embodiment of the present invention, the microarray has attached on its surface at least one nucleic acid probe comprising a sequence of the 16S rRNA, preferably of mycoplasma, most preferred the nucleic acid probe contains a sequence as defined by SEQ ID NO: 1.

For commercial convenience, the microrray of the present invention which has attached on its surface at least one nucleic acid probe representing a gene of said cell culture and at least one nucleic acid probe representing a gene of said microorganism for specific detection and identification of the microorganism in a cell culture may be packaged in the form of a kit.

Typically, such a kit additionally contains at least one type of primers specific for one or more microorganisms. Reagents for performing a nucleic acid reverse transcription reaction may also be included, for example, buffers, additional primers, optionally labelled nucleotide triphosphates, enzymes, a positive template control, etc. The components of the kit are packaged together in a common container, optionally including instructions for performing a specific embodiment of the inventive methods. Other optional components may also be included in the kit, e.g., an oligonucleotide tagged with a label suitable for use as an assay probe, and/or reagents or means for detecting the label.

In a further embodiment, the present invention refers to a method for analyzing the effect of a microorganism contamination on the gene expression of eukaryotic cells, the method comprising:
(a) providing a microarray which has attached on its surface
   (a.1) at least one nucleic acid probe representing a gene of a eukaryotic cell and
   (a.2) at least one nucleic acid probe representing a gene of a microorganism,
(b) preparing nucleic acid targets from the cell culture by means of a primer mixture suitable for amplifying said at least one gene of a eukaryotic cell and said at least one gene of a microorganism,
(c) contacting the microarray of step (a) with the nucleic acid targets of step (b) to permit selective hybridization between the nucleic acid targets and their complementary nucleic acid probes on the microarray
(d) detecting said hybridization thereby detecting the expression of genes specific a eukaryotic cells and detecting said microorganism contamination and
(e) comparing the gene expression of contaminated eukaryotic cells with the gene expression of non-contaminated eukaryotic cells, wherein an altered expression of one or more genes is indicative of an effect of the microorganism on the expression of said genes.

Furthermore, the present invention refers to a method for testing the suitability of a culture of eukaryotic cells for gene expression profiling by detecting the absence or presence of a microorganism, the method comprising:
(a) providing a microarray which has attached on its surface
   (a.1) at least one nucleic acid probe representing a gene of a eukaryotic cell and
   (a.2) at least one nucleic acid probe representing a gene of said microorganism,
(b) preparing nucleic acid targets from the cell culture by means of a primer mixture suitable for amplifying said at least one gene of a eukaryotic cell and said at least one gene of a microorganism,
(c) contacting the microarray of step (a) with the nucleic acid targets of step (b) to permit selective hybridization between the nucleic acid targets and their complementary nucleic acid probes on the microarray, and
(d) detecting said hybridization thereby detecting the absence or presence of a microorganism, wherein the absence indicates the suitability of said cell culture for gene expression profiling.

Preferably, the method comprises an additional step:
(e) using the microarray of step c) for gene expression profiling provided the absence of a microorganism has been detected.

The methods of the present invention, in particular the compounds required for performing the methods, e.g. primers and microarrays, are useful whenever a high throughput screening for microorganism contamination of eukaryotic cells to be used for gene expression profiling is desired.

Thus, the present invention refers to the use of a primer comprising the nucleic acid sequence complementary to the target region of a microorganism for detecting a microorganism contamination in a culture of eukaryotic cells to be used for gene expression profiling.

Furthermore, the present invention refers to the use of a primer comprising the nucleic acid sequence complementary to the target region of a microorganism for analyzing the effect of a microorganism contamination on the gene expression of eukaryotic cells.

Furthermore, the present invention refers to the use of a primer comprising the nucleic acid sequence complementary to the target region of a microorganism for testing the suitability of a culture of eukaryotic cells for gene expression profiling by detecting the absence or presence of a microorganism.

For the uses of the primer of the present invention described above it is preferred that the target region of a microorganism is 16S rRNA, preferably the 16S rRNA of Mycoplasma.

In a most preferred embodiment of the present invention, the gene expression profiling in the above described uses is done by the use of a microarray which has attached on its surface
(a.1) at least one nucleic acid probe representing a gene of a eukaryotic cell and
(a.2) at least one nucleic acid probe representing a gene of a microorganism.

In another embodiment of the present invention, the microarray which has attached on its surface
(a.1) at least one nucleic acid probe representing a gene of a eukaryotic cell and
(a.2) at least one nucleic acid probe representing a gene of a microorganism
can be used for detecting a microorganism contamination in a culture of eukaryotic cells which should be used for gene expression profiling.

In addition, the present invention refers to the use of a culture of eukaryotic cells, an extract or fraction thereof, for gene expression profiling, characterized in that said culture of eukaryotic cells is tested for suitability for gene expression by a method comprising:
(a) providing a microarray which has attached on its surface
   (a.1) at least one nucleic acid probe representing a gene of a eukaryotic cell and
   (a.2) at least one nucleic acid probe representing a gene of said microorganism,
(b) preparing nucleic acid targets from the cell culture by means of a primer mixture suitable for amplifying said at least one gene of a eukaryotic cell and said at least one gene of said microorganism,
(c) contacting the microarray of step (a) with the nucleic acid targets of step (b) to permit selective hybridization between the nucleic acid targets and their complementary nucleic acid probes on the microarray, and
(d) detecting said hybridization thereby detecting the absence or presence of a microorganism, wherein the absence indicates the suitability of said cell culture for gene expression profiling.

### DESCRIPTION OF THE DRAWING

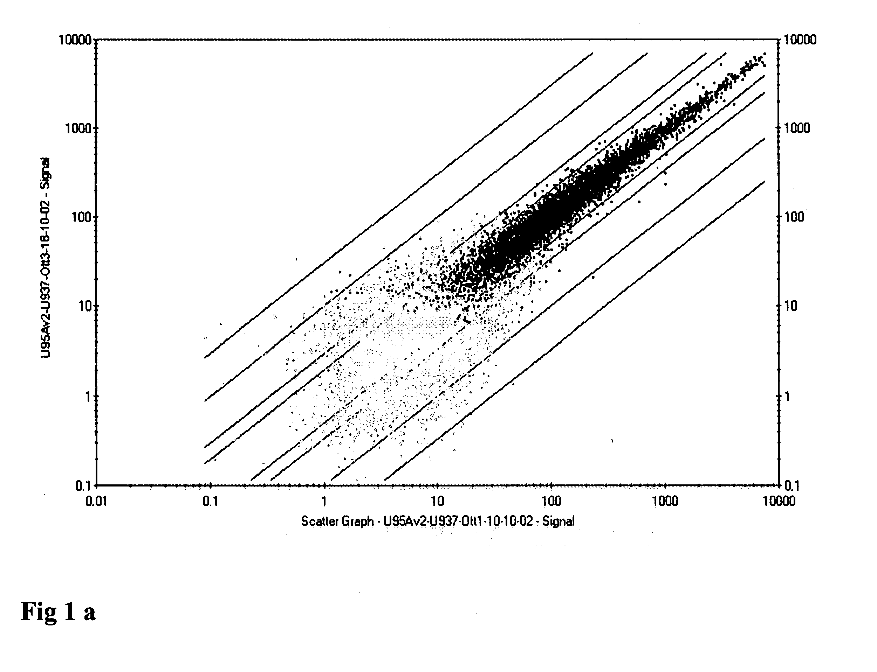

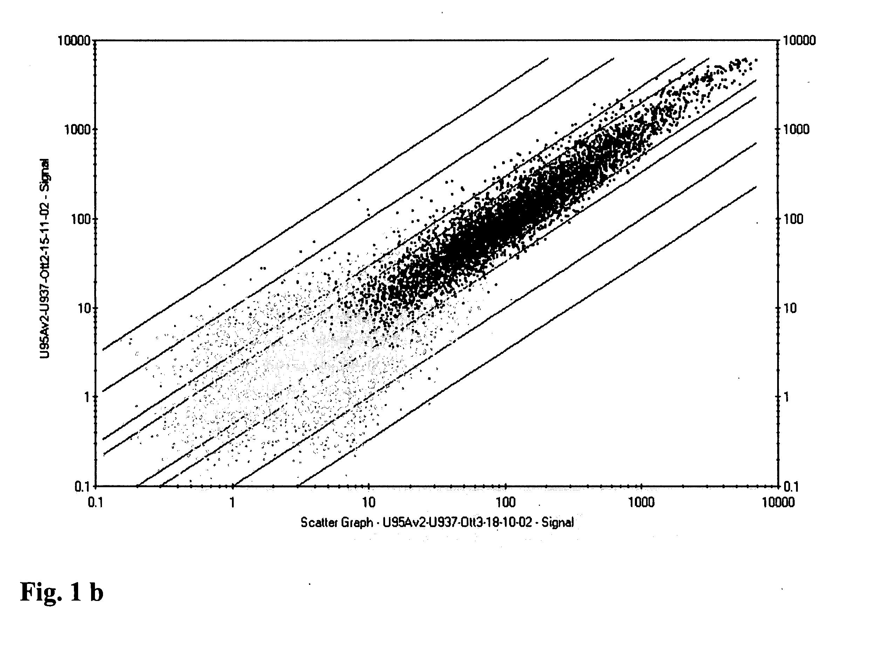

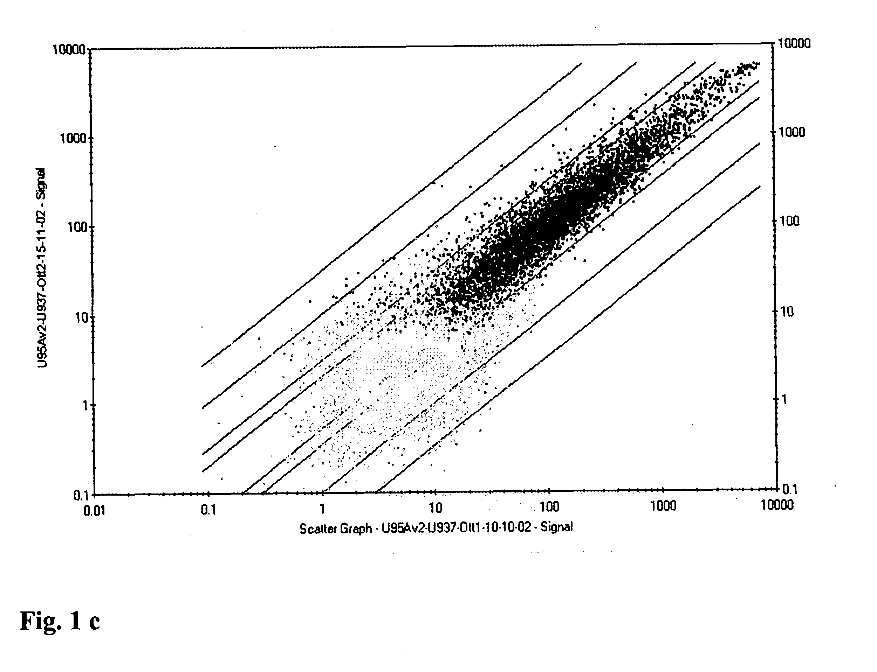

The drawing shows a scatter graph analysis of the gene expression profile performed with cell cultures which are not contaminated with Mycoplasma (Fig. 1a) and scatter graph analyses of the gene expression profile performed with cell cultures which are contaminated with Mycoplasma (Fig. 1b and c).

The invention is further illustrated by the following example:

### EXAMPLE

### Cell lines.

Parvovirus H-1 sensitive human U937, U937 A and virus-resistant RU 1 to 4 sublines from U937 A ( Lopez-Guerrero, J.A. et al., Blood. 1997, Vol. 89(5):1642-53) were passaged in RPMI 1640 medium with 10% heat-inactivated fetal calf serum, 2 mM glutamina and penicillin/ streptomycin at 37 °C and 5% CO₂.

### Isolation of total RNA for expression profiling.

Throughout the experiments, cells in the logarithmic growth phase at a density of 225.000 to 400.000 cells/ ml were harvested, washed once in PBS at room temperature, and extracted for RNA using the Rneasy Mini kit (Qiagen, Hilden, Germany) following supplier's instructions. To reduce biological variance, RNAs from three independently grown unstimulated cell batches were pooled (a '3-pool) and hybridised to one chip, and the experiment was repeated three times. Amounts of RNA were typically 20 µg. Following essentially Affymetrix instructions was converted into biotin-labeled fragmented cRNA ready for hybridisation.

### Profiling of labeled cRNA on Affymetrix HG-U95A chips.

Five µg of fragmented cRNA in 80 µl of hybridisation buffer (Affymetrix protocol) were hybridised overnight at 45°C to Affymetrix T2 Test chips; evaluation of these chips showed in each case that the initial RNA was undegraded. 240 µl of cRNA in hybridisation buffer were subsequently hybridised to U95A chips which contain about 12 600 sequences of the human genome. Scans of the chips were evaluated by the Affymetrix MAS 5.0 software. The default average target intensity on a chip was set to 100. The reproducibility of three identical cell lines resulted in a correlation coefficient of r=0,96, about 45% of the sequences on a HG-95A chip gave a present call.

### Examples of 16S rRNA and 23S rRNA Sequences which can be used as primers and nucleic acid probes for the detection of a mycoplasma contamination

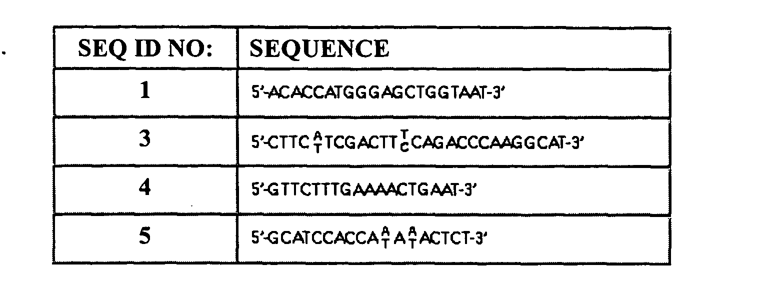

SEQ ID NO: refers to a sequence of the 16S rRNA gene of a variety of mycoplasma species. SEQ ID NOs:3 and 5 refer to a sequence within the 23S rRNA gene of a variety of mycoplasma species, SEQ ID NO:4 refers to a so-called spacer region between the 16S and the 23S rRNA genes of a variety of mycoplasma species. It is understood by skilled artisan that, in order to hybridize to and amplify the desired and correct strand, nucleic acids containing sequences which are complementary to the SEQ ID NOs above are also included in the present invention.

## Claims

1. Method for detecting a microorganism contamination in a culture of eukaryotic cells to be used for gene expression profiling, the method comprising:
(a) providing a microarray which has attached on its surface
(a.1) at least one nucleic acid probe representing a gene of a eukaryotic cell and
(a.2) at least one nucleic acid probe representing a gene of a microorganism,
(b) preparing nucleic acid targets from said culture by means of a primer mixture suitable for amplifying said at least one gene of a eukaryotic cell and said at least one gene of a microorganism,
(c) contacting the microarray of step (a) with the nucleic acid targets of step (b) to permit selective hybridization between the nucleic acid targets and their complementary nucleic acid probes on the microarray
and
(d) detecting said hybridization thereby detecting a microorganism contamination and, optionally, detecting the expression of genes specific for the eukaryotic cell.

2. The method according to claim 1, the method comprising an additional step:
(e) comparing the gene expression of contaminated eukaryotic cells with the gene expression of non-contaminated eukaryotic cells.

3. The method according to claim 1 or 2, wherein the microorganism belongs to the class of mollicutes.

4. The method according to any of claims 1 to 3, wherein the mollicutes comprise the genera Mycoplasma, Ureaplasma, Acholeplasma and Spiroplasma.

5. The method according to any of claims 1 to 4, wherein the genus is Mycoplasma.

6. The method according to any of claims 1 to 5, wherein the nucleic acid probe representing a gene of the microorganism and the primer specific for said microorganism comprises a nucleic acid sequence of the 16S or 23S rRNA gene, preferably the 16S or 23S rRNA of mycoplasma.

7. The method according to claim 6, wherein said nucleic acid sequence of contains a sequence as defined by SEQ ID NOs: 1, 3, 4 or 5.

8. The method according to any of claims 1 to 7, wherein the preparing of a nucleic acid target sample in step (b) of claim 1 includes an in vitro transcription reaction.

9. The method according to claim 8, wherein the in vitro transcription is mediated by the use of primers containing the sequence of the T7 promoter, the T3 promoter or the SP6 promoter.

10. The method according to claim 9, wherein the promoter is the T7 promoter as defined by SEQ ID NO:2.

11. The method according to any of claims 1 to 10, wherein the eukaryotic cells are mammalian cells.

12. The method according to claim 11, wherein the mammalian cells are human cells.

13. A microarray which has attached on its surface
(a.1) at least one nucleic acid probe representing a gene of a eukaryotic cell and
(a.2) at least one nucleic acid probe representing a gene of a microorganism.

14. The microarray according to claim 13, wherein the nucleic acid probe in (a.2) comprises a sequence of the 16S rRNA gene, preferably of mycoplasma.

15. The microarray according to claim 14, wherein the sequence is defined by SEQ ID NO:1.

16. A diagnostic kit for detecting the presence of a microorganism in a cell culture, the kit containing a microarray as defined in any of claims 13 to 15, a suitable primer mixture, suitable enzymes, optionally labelled rNTPs, and a positive template control.

17. Method for analyzing the effect of a microorganism contamination on the gene expression of eukaryotic cells, the method comprising:
(a) providing a microarray which has attached on its surface
(a.1) at least one nucleic acid probe representing a gene of a eukaryotic cell and
(a.2) at least one nucleic acid probe representing a gene of a microorganism,
(b) preparing nucleic acid targets from the cell culture by means of a primer mixture suitable for amplifying said at least one gene of a eukaryotic cell and said at least one gene of a microorganism,
(c) contacting the microarray of step (a) with the nucleic acid targets of step (b) to permit selective hybridization between the nucleic acid targets and their complementary nucleic acid probes on the microarray
(d) detecting said hybridization thereby detecting the expression of genes specific a eukaryotic cells and detecting said microorganism contamination
and
(e) comparing the gene expression of contaminated eukaryotic cells with the gene expression of non-contaminated eukaryotic cells, wherein an altered expression of one or more genes is indicative of an effect of the microorganism on the expression of said genes.

18. Method for testing the suitability of a culture of eukaryotic cells for gene expression profiling by detecting the absence or presence of a microorganism, the method comprising:
(a) providing a microarray which has attached on its surface
(a.1) at least one nucleic acid probe representing a gene of a eukaryotic cell and
(a.2) at least one nucleic acid probe representing a gene of said microorganism,
(b) preparing nucleic acid targets from the cell culture by means of a primer mixture suitable for amplifying said at least one gene of a eukaryotic cell and at said least one gene of a microorganism,
(c) contacting the microarray of step (a) with the nucleic acid targets of step (b) to permit selective hybridization between the nucleic acid targets and their complementary nucleic acid probes on the microarray, and
(d) detecting said hybridization thereby detecting the absence or presence of a microorganism, wherein the absence indicates the suitability of said cell culture for gene expression profiling.

19. The method according to claim 23, wherein the method comprises an additional step:
(f) using the microarray of step c) for gene expression profiling provided the absence of a microorganism has been detected.

20. Use of a primer comprising the nucleic acid sequence complementary to the target region of a microorganism for detecting a microorganism contamination in a culture of eukaryotic cells to be used for gene expression profiling.

21. Use of a primer comprising the nucleic acid sequence complementary to the target region of a microorganism for analyzing the effect of a microorganism contamination on the gene expression of eukaryotic cells.

22. Use of a primer comprising the nucleic acid sequence complementary to the target region of a microorganism for testing the suitability of a culture of eukaryotic cells for gene expression profiling by detecting the absence or presence of a microorganism.

23. The use according to any of claims 20 to 22, wherein the target region of a microorganism is 16S rRNA, preferably the 16S rRNA of Mycoplasma.

24. The use according to any of claims 20 to 23, wherein the gene expression profiling is done by using a microarray as defined in any of claims 13 to 15.

25. Use of a microarray as defined in any of claims 13 to 15 for detecting a microorganism contamination in a culture of eukaryotic cells to be used for gene expression profiling.

26. Use of a culture of eukaryotic cells, an extract or fraction thereof, for gene expression profiling, **characterized in that** said culture of eukaryotic cells is tested for suitability for gene expression by a method as defined in claim 18 or 19.
